# EUROPEAN PATENT APPLICATION

(11) **EP 4 495 254 A1**
(43) Date of publication of application: **22.01.2025**
(21) Application number: 24196468.3
(22) Date of filing: 26.05.2021
(51) Int. Cl.: C12Q 1/6869, G16B 30/10

(54) **SEQUENCE ALIGNMENT SYSTEMS AND METHODS TO IDENTIFY SHORT MOTIFS IN HIGH-ERROR SINGLE-MOLECULE READS**

(30) Priority: 28.05.2020 US 202063030931 P
(62) Divisional of application: 21729468.5
(71) Applicant: F. Hoffmann-La Roche AG, 4070 Basel (CH); Roche Diagnostics GmbH, 68305 Mannheim (DE)
(72) Inventor: LAU, Bayo, Pleasanton, 94588 (US); KUKRICAR, Miroslav, Pleasanton, 94588 (US)
(74) Representative: Merkel, Patrick

(57) **Abstract**

Described herein is a novel alignment method which leverages multi-stage secondary analysis, with each stage progressively reducing the amount of data to be analyzed in the next stage(s), but increasing exhaustiveness of the search on the remaining data received from previous stage(s). This way, less noisy alignments can be quickly identified from the initially large data-pools in early stage(s), while very noisy alignments can be identified equally fast from smaller data-pools in latter stage(s) of computation, thus maintaining target sensitivity while reducing overall compute times.

## Description

### CROSS REFERENCE TO RELATED APPLICATIONS

None.

### INCORPORATION BY REFERENCE

All publications and patent applications mentioned in this specification are herein incorporated by reference to the same extent as if each individual publication or patent application was specifically and individually indicated to be incorporated by reference.

### FIELD

Embodiments of the invention relate generally to aligning sequences, and more specifically to efficiently identifying short motifs in long sequences with high error rates.

### BACKGROUND

Commercial sequencing systems generally produce either short reads having low error rates (i.e., Illumina sequencers) or long reads having high error rates (i.e., Pacific Biosciences sequencers). Consequently, most sequence alignment tools have been developed and optimized for both these use cases: (1) identifying short motifs from short reads having low error rates or (2) identifying long motifs from long reads having high error rates. However, in certain assays it is desirable to be able to identify short motifs from long sequences having high error rates.

### SUMMARY OF THE DISCLOSURE

The present invention relates generally to aligning sequences, and more specifically to efficiently identifying short motifs in long sequences with high error rates.

In some embodiments, a method for aligning sequence reads to a reference sequence is provided. The method can include aligning a first set of sequence reads from an entire population of sequence reads to a reference sequence with a Burrows-Wheeler transform using a first seed length, wherein the first seed length is selected based on an error rate of the sequence reads; masking the first set of sequence reads such that the entire population of sequence reads comprises a subset of masked sequence reads and unmasked sequence reads; aligning a second set of sequence reads from the unmasked sequence reads to the reference sequence with the Burrow-Wheeler transform using a second seed length, wherein the second seed length is smaller or shorter than the first seed length, in order to achieve greater sensitivity of the second alignment step; and determining an alignment of the sequence reads to the reference sequence based on the first set of sequence reads and the second set of sequence reads.

In some embodiments, the method further includes iteratively masking and aligning additional sets of sequence reads with each subsequent set of reads having a greater seed length, and determining the alignment of the sequence reads with the additional sets of sequence reads.

In some embodiments, the first seed length is less than 10 bases. In some embodiments, the first seed length is less than 5 bases. In some embodiments, the first seed length is 4 bases.

In some embodiments, the error rate of the sequence reads is at least 5%. In some embodiments, the error rate of the sequence reads is at least 10%. In some embodiments, the error rate of the sequence reads is at least 15%.

In some embodiments, the sequence reads are sequenced from a plurality of concatamers, wherein each concatamer is formed of oligonucleotide sequences that have been joined together, wherein the oligonucleotide sequences correspond to a plurality of loci from a set of chromosomes. In some embodiments, the set of chromosomes comprises chromosome 13, 18, 22, X, and Y. In some embodiments, the set of chromosomes is selected from the group consisting of chromosome 13, 18, 22, X, and Y. In some embodiments, the method further includes calculating a frequency each loci is found in the sequence reads.

In some embodiments, a method for aligning sequence reads to a reference sequence is provided. The method can include aligning a first set of sequence reads from an entire population of sequence reads to a reference sequence with a Burrows-Wheeler transform using a first set of sensitivity parameters, wherein the first set of sensitivity parameters is selected based on an error rate of the sequence reads; masking the first set of sequence reads such that the entire population of sequence reads comprises a subset of masked sequence reads and unmasked sequence reads; aligning a second set of sequence reads from the unmasked sequence reads to the reference sequence with the Burrow-Wheeler transform using a second set of sensitivity parameters, wherein the second set of sensitivity parameters results in a higher sensitivity than the first set of sensitivity parameters; determining an alignment of the sequence reads to the reference sequence based on the first set of sequence reads and the second set of sequence reads.

In some embodiments, the method further includes iteratively masking and aligning additional sets of sequence reads with each subsequent set of reads having a set of sensitivity parameters that results in higher sensitivity, and determining the alignment of the sequence reads with the additional sets of sequence reads.

In some embodiments, the sensitivity parameters are selected from the group consisting of seed generation, chaining and filtering, and thresholding.

In some embodiments, a computer product includes a computer readable medium storing a plurality of instructions for controlling a computer system to perform an operation of any of the methods above.

In some embodiments, a system includes the computer product above and one or more processors for executing instructions stored on the computer readable medium.

In some embodiments, a system includes means for performing any of the above methods.

In some embodiments, a system includes one or more processors configured to perform any of the above methods.

In some embodiments, a system includes modules that respectively perform the steps of any of the above methods.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 is a block diagram illustrating one embodiment of a computer system configured to implement one or more aspects of the present invention.
FIG. 2 illustrates that in one embodiment sample reads in an NIPT secondary analysis can be concatamers of short NIPT indices that correspond to a unique locus on a chromosome of interest (i.e, 13, 18, 22, X, Y), and these NIPT indices in the sample reads are aligned to an index database to determine the frequencies of the NIPT indices in the sample reads.
FIG. 3 illustrates a flow chart of an embodiment of a multi-stage alignment process.
FIG. 4 compares key cost/performance metrics between traditional BWA aligner (blue) and an embodiment of an optimized aligner (orange) described herein.

### DETAILED DESCRIPTION

A nanopore based sequencing system can generate vast amounts of long read sequencing data, depending on the number of nanopore based sensors that are fabricated in the sensing chip. In some embodiments, each sensing chip can have millions of cells, each with a nanopore sensor. One advantage of using a nanopore sequencer is the ability to generate long reads. However, the current raw read accuracy of nanopore sequencers is generally lower (i.e., between 80 to 95%) than established short read technologies (i.e., accuracy greater than 99%).

To take advantage of the long read capability of a nanopore sequencer, relatively short target sequences can be concatemerized into a longer linear sequence that can be efficiently sequenced in the nanopore sequencer. The concatemers can be sequenced in parallel by the nanopore sequencer to generate a consensus sequence with the desired degree of accuracy (i.e., greater than 99%, 99.9%, or 99.99%, for example). In order to generate the consensus sequence, the sequence fragments need to be aligned.

Although the alignment methods and systems are described in the context of a nanopore sequencer, other sequences generated by other types of sequencing devices can also be aligned using the alignment methods and systems described herein. For example, Non-limiting examples of sequence assays that are suitable for use with the methods disclosed herein include nanopore sequencing (US Pat. Publ. Nos. 2013/0244340, 2013/0264207, 2014/0134616, 2015/0119259 and 2015/0337366), Sanger sequencing, capillary array sequencing, thermal cycle sequencing (Sears et al., Biotechniques, 13:626-633 (1992)), solid-phase sequencing (Zimmerman et al., Methods Mol. Cell Biol., 3:39-42 (1992)), sequencing with mass spectrometry such as matrix-assisted laser desorption/ionization time-of-flight mass spectrometry (MALDI-TOF/MS; Fu et al., Nature Biotech., 16:381-384 (1998)), sequencing by hybridization (Drmanac et al., Nature Biotech., 16:54-58 (1998), and NGS methods, including but not limited to sequencing by synthesis (e.g., HiSeq^{™}, MiSeq^{™}, or Genome Analyzer, each available from Illumina), sequencing by ligation (e.g., SOLiD^{™}, Life Technologies), ion semiconductor sequencing (e.g., Ion Torrent^{™}, Life Technologies), and SMRT^{®} sequencing (e.g., Pacific Biosciences).

Commercially available sequencing technologies include: sequencing-by-hybridization platforms from Affymetrix Inc. (Sunnyvale, Calif.), sequencing-by-synthesis platforms from Illumina/Solexa (San Diego, Calif.) and Helicos Biosciences (Cambridge, Mass.), sequencing-by-ligation platform from Applied Biosystems (Foster City, Calif.). Other sequencing technologies include, but are not limited to, the Ion Torrent technology (ThermoFisher Scientific), and nanopore sequencing (Genia Technology from Roche Sequencing Solutions, Santa Clara, Cal.); and Oxford Nanopore Technologies (Oxford, United Kingdom).

While alignment analysis is not a new topic, certain sequencing applications present a challenge. For example, the noninvasive prenatal testing (NIPT) assay presents a case that is not well developed in the world of bioinformatics. Identification of short motifs, for example in one embodiment NIPT indices of 40 base-pair, from long and noisy read sequences (in one embodiment concatemer nanopore reads with 1000-5000 base-pairs and 10-20% of error) is a new consideration to algorithm design because of the needs to satisfy both compute time / resource constraints as well as to meet strict sensitivity and specificity requirements of the assay. Most existing alignment tools and methods are designed for short motifs sequenced at low error rates (e.g., Illumina sequencers) and for long motifs with high error rate (e.g., Pacific Bioscience sequencers): in fact, the NIPT assay's use-case of short motifs and high error rate is exactly the weakness of these approaches, which suffer in terms of sensitivity, compute resource or both.

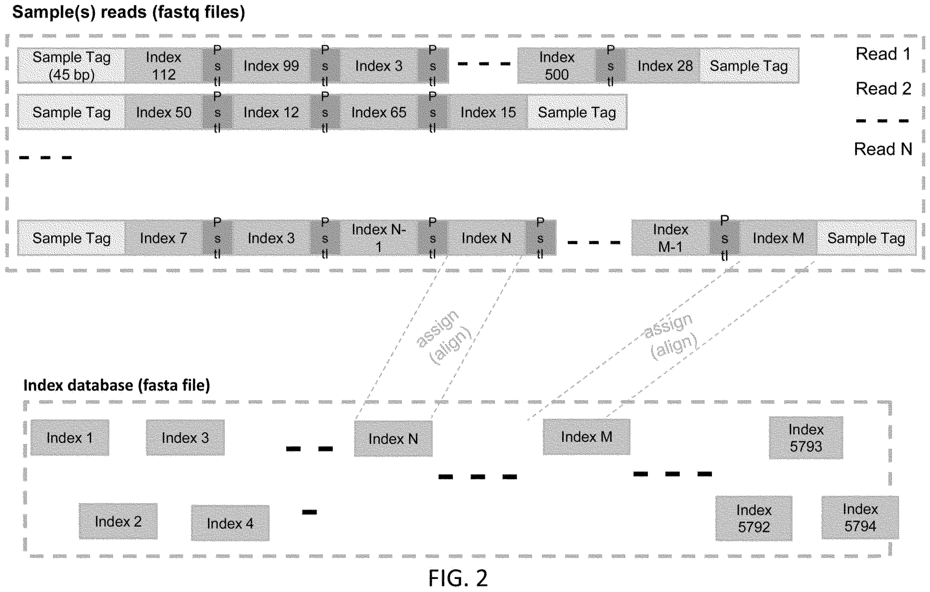

As a concrete consideration, genomic aligners are designed for cases wherein only a few very long reference sequences (e.g., chromosomes of millions base-pairs) are used as the alignment target and each read sequence is expected to be aligned uniquely to one single target locus. In contrast, in one embodiment the NIPT assay probes biological samples with artificial short sequence motifs (NIPT Indices), cleaves them with a restriction enzyme (i.e. Pstl) and concatenates them into a long template (i.e., greater than 100, 200, 300, 400, 500, 600, 700, 800, 900, 1000, 2000, 3000, 4000, 5000, 6000, 7000, 8000, 9000, or 1000 bases), containing non-genomic short oligos, and sequences the template as long, high error reads (i.e. at least 1, 2, 3, 4, 5, 6, 7, 8, 9, 10, 11, 12, 13, 14, 15, 16, 17, 18, 19, or 20 percent). The NIPT assay can have over 5000 very short reference sequences (NIPT indices of 40 base-pairs) and each concatemer read sequence aligns as a set of non-overlapping segments to multiple targets at up to 20% error rate, as shown in FIG. 2. The goal of secondary analysis is to identify those indices in concatemer reads, i.e. to find frequency of each database index in given patient sample. Index frequencies are then sent to a tertiary analysis module (i.e., "Forte") which builds statistical model from chromosomal frequencies and establishes screening test outcome (probability), recommendation for clinician, etc.

Similar reasons, as well as low noise resilience, are applicable against analytical tools covering other classes of alignment problems, making them unfit or non-optimal for the type of the alignment problems found in certain types of sequencing based assays. For example, short motifs, and well-defined ground truth (e.g., set of 5000+ target NIPT indices without possible allelic polymorphisms and/or other inconsistencies typical for genomic analysis) would make this type of problem seemingly fit for an alignment-free approach, e.g. using sequence k-mer seeding/hashing. However, the presence of high noise in reads would require very short k-mer length to maintain high sensitivity. For example, internal data showed in practice that changing k-mer length from 4 base-pairs to 5 base-pairs could lead to loss of sensitivity of 10% or more, depending on the read error-rate. Short(er) k-mers also create additional computational burden (typically complexity is O(n²), where n is number of k-mers, inversely proportional to k-mer length).

We conceived a novel alignment method which leverages multi-stage secondary analysis, with each stage progressively reducing the amount of data to be analyzed in the next stage(s), but increasing exhaustiveness of the search on the remaining data received from previous stage(s). This way, less noisy alignments can be quickly identified from the initially large data-pools in early stage(s), while very noisy alignments can be identified equally fast from smaller data-pools in latter stage(s) of computation, thus maintaining target sensitivity while reducing overall compute times.

We found that the alignment method requires a non-trivial computational implementation, because we showed that a naive, non-optimized implementation of the alignment method would lead to additional auxiliary data/compute cost (which negates the alignment method's benefit) and also potential incompatibility with downstream processing.

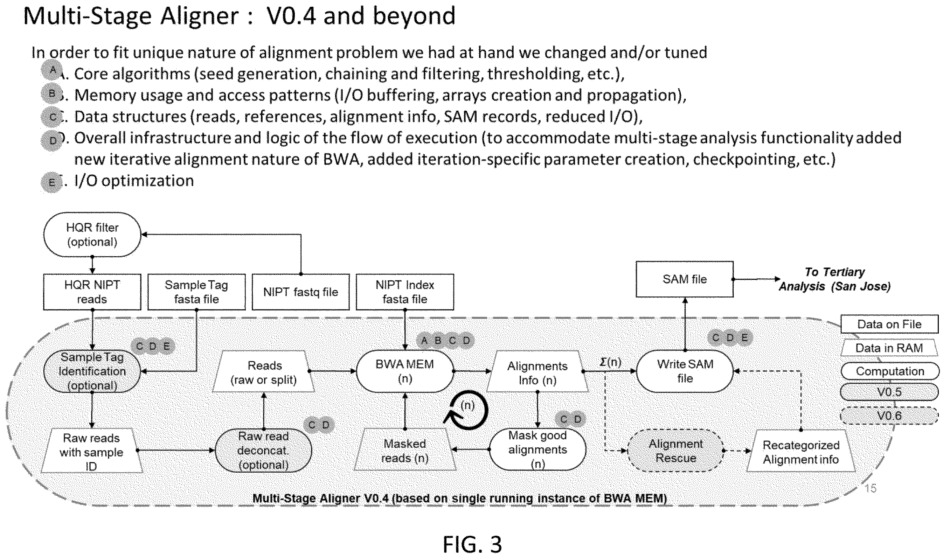

To address all these, we implemented our alignment method as a highly optimized and concise extension to the industry-standard BWA-MEM, a well-established open-source genomic alignment tool under the Apache license. We had to change and tune its core algorithms, data structures and context-dependent optimizations to fit the unique nature of alignment problem we had at hand for the NIPT assay. We also had to change its overall infrastructure to accommodate our multi-stage analysis functionality. More specifically, the following parts of BWA-MEM were changed or tuned: core algorithms (seed generation, chaining and filtering, thresholding, etc.), data structures (reads, references, alignment info, SAM records, reduced I/O), memory usage and access patterns (I/O buffering, arrays creation and propagation), and the overall infrastructure and logic of the flow of execution (to accommodate multi-stage analysis functionality we added a new iterative alignment procedure to BWA, added iteration-specific parameter creation, checkpointing, etc.). A flow chart of the modified and optimized workflow of BWA-MEM is presented in FIG. 3.

For example, in some embodiments the seed parameter is reduced from a 19 base or base pair kmer (i.e., BWA-MEM default) to a 4 base or base pair kmer. The size of the reduced kmer is based on the error rate. If the error rate of the sequence reads is decreased, then the seed length can be increased to reduce the computation time. An increased first pass seed size, as compared to the second pass seed size, allows the first pass to quickly identify oligo sequences that match an NIPT index (but with lower sensitivity), which allows these sequences to be removed from the unaligned sequence pool, which can be processed using higher sensitivity parameters in the next pass, such as a smaller seed size (seed generation, chaining and filtering, thresholding, etc.). The process of seeding involves converting the kmer to an integer value (i.e., each base type has a particular value and the kmer is the sum of the base values). These integer values can be much more easily and quickly compared with the corresponding kmer integer values from the reference NIPT index than trying to match the kmer strings themselves.

Along with seed generation, chaining can be tuned to improve performance. Chaining is the process of finding groups of seeds, called chains, that are overlapping seeds or seeds that are co-linear and close to each other. The chains can be identified by various grouping algorithms, such as the pigeon hole method or greedy clustering algorithm.

This project was exciting because we have developed a novel use for arguably the most popular bioinformatics tool of today. Importance of this project lies in the fact that it has enabled nanopore sequencing based NIPT assay secondary analysis to be rapidly completed (i.e., less than 1, 2, 3, 4, 5, or 6 hours). It also enabled analysis on virtually any kind of commodity hardware, a stark contrast to initial project estimates for very expensive high-end computing hardware.

Work on this project also lays ground for similar algorithmic approaches in other areas (i.e. sequencing based assays) where long reads carrying short and noisy motifs could be leveraged, but lack highly optimized tools and algorithms, for example, inference of splice-variant events in RNA-seq isoforms, meta-transcriptomics, etc.

One of the impacts of the optimized alignment method is in direct cost reduction for the final product. Initial estimates before optimization for hardware configuration required for secondary analysis for the NIPT assay were predicting compute workstation with 256-512 GB of RAM and 32-40 CPU cores. The optimized alignment method reduced compute time by 2X (reduction in CPU cores) and expected RAM memory usage by 98%. It is hard to estimate exact cost-reduction impact, but it is fair to say it will be in tens of thousands of dollars per each installed configuration, potentially saving up to 50% of computing hardware cost.

A second impact is reduction in compute time. By reducing compute time, it was also possible to fit per-run cycle analysis into a 1-hour time allotment, so that secondary analysis on previous run cycle can be performed while instrument is sequencing next run cycle, effectively making secondary run analysis results ready within one hour after sequencing experiment completion. This significant product requirement was not feasible and at risk with the previous, unoptimized approach.

The alignment method described herein is particularly suited for identifying short motifs from long, high error reads. In some embodiments, a short motif is less than about 50, 60, 70, 80, 90, 100, 200, 300, 400, 500, 600, 700, 800, 900, or 1000 bases or base pairs. In some embodiments, a high error read is a raw read with an error rate of greater than about 1, 2, 3, 4, 5, 6, 7, 8, 9, 10, 11, 12, 13, 14, 15, 16, 17, 18, 19, or 20%. In some embodiments, long reads have at least 100, 200, 300, 400, 500, 600, 700, 800, 900, 1000, 2000, 3000, 4000, 5000, 6000, 7000, 8000, 9000, or 10000 bases or base pairs. In some embodiments, the algorithm can be applied to shorter length high error reads, depending on the output of the sequencer. For example, shorter reads can be less than 1000, 900, 800, 700, 600, or 500 bases.

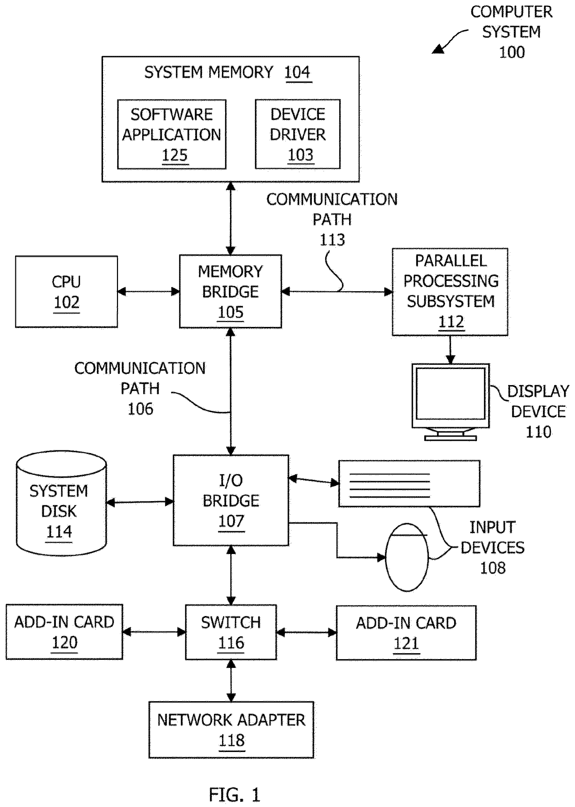

The alignment methods and algorithms described herein can be implemented on a computer system. For example, FIG. 1 is a block diagram illustrating one embodiment of a computer system **100** configured to implement one or more aspects of the present invention. As shown, computer system **100** includes, without limitation, a central processing unit (CPU) **102** and a system memory **104** coupled to a parallel processing subsystem **112** via a memory bridge **105** and a communication path **113.** Memory bridge **105** is further coupled to an I/O (input/output) bridge **107**via a communication path **106,** and I/O bridge **107** is, in turn, coupled to a switch **116.**

In operation, I/O bridge **107** is configured to receive user input information from input devices **108** (e.g., a keyboard, a mouse, a video/image capture device, etc.) and forward the input information to CPU **102** for processing via communication path **106** and memory bridge **105.** In some embodiments, the input information is a live feed from a camera/image capture device or video data stored on a digital storage media on which object detection operations execute. Switch **116** is configured to provide connections between I/O bridge **107** and other components of the computer system **100,** such as a network adapter **118** and various add-in cards **120** and **121.**

As also shown, I/O bridge **107** is coupled to a system disk **114** that may be configured to store content and applications and data for use by CPU **102**and parallel processing subsystem **112.** As a general matter, system disk **114** provides non-volatile storage for applications and data and may include fixed or removable hard disk drives, flash memory devices, and CD-ROM (compact disc read-only-memory), DVD-ROM (digital versatile disc-ROM), Blu-ray, HD-DVD (high definition DVD), or other magnetic, optical, or solid state storage devices. Finally, although not explicitly shown, other components, such as universal serial bus or other port connections, compact disc drives, digital versatile disc drives, film recording devices, and the like, may be connected to I/O bridge **107** as well.

In various embodiments, memory bridge **105** may be a Northbridge chip, and I/O bridge **107** may be a Southbrige chip. In addition, communication paths **106** and **113,** as well as other communication paths within computer system **100,** may be implemented using any technically suitable protocols, including, without limitation, AGP (Accelerated Graphics Port), HyperTransport, or any other bus or point-to-point communication protocol known in the art.

In some embodiments, parallel processing subsystem **112** comprises a graphics subsystem that delivers pixels to a display device **110** that may be any conventional cathode ray tube, liquid crystal display, light-emitting diode display, or the like. In such embodiments, the parallel processing subsystem **112** incorporates circuitry optimized for graphics and video processing, including, for example, video output circuitry. Such circuitry may be incorporated across one or more parallel processing units (PPUs) included within parallel processing subsystem **112.** In other embodiments, the parallel processing subsystem **112** incorporates circuitry optimized for general purpose and/or compute processing. Again, such circuitry may be incorporated across one or more PPUs included within parallel processing subsystem **112** that are configured to perform such general purpose and/or compute operations. In yet other embodiments, the one or more PPUs included within parallel processing subsystem **112** may be configured to perform graphics processing, general purpose processing, and compute processing operations. System memory **104** includes at least one device driver **103** configured to manage the processing operations of the one or more PPUs within parallel processing subsystem **112.** The system memory **104** also includes a software application **125** that executes on the CPU **102** and may issue commands that control the operation of the PPUs.

In various embodiments, parallel processing subsystem **112** may be integrated with one or more other the other elements of FIG. 1 to form a single system. For example, parallel processing subsystem **112** may be integrated with CPU **102** and other connection circuitry on a single chip to form a system on chip (SoC).

It will be appreciated that the system shown herein is illustrative and that variations and modifications are possible. The connection topology, including the number and arrangement of bridges, the number of CPUs **102,** and the number of parallel processing subsystems **112,** may be modified as desired. For example, in some embodiments, system memory **104** could be connected to CPU **102** directly rather than through memory bridge **105,** and other devices would communicate with system memory **104** via memory bridge **105** and CPU **102.** In other alternative topologies, parallel processing subsystem **112** may be connected to I/O bridge **107** or directly to CPU **102,** rather than to memory bridge **105.** In still other embodiments, I/O bridge **107** and memory bridge **105** may be integrated into a single chip instead of existing as one or more discrete devices. Lastly, in certain embodiments, one or more components shown in FIG. 1 may not be present. For example, switch **116** could be eliminated, and network adapter **118** and add-in cards **120, 121** would connect directly to I/O bridge **107.**

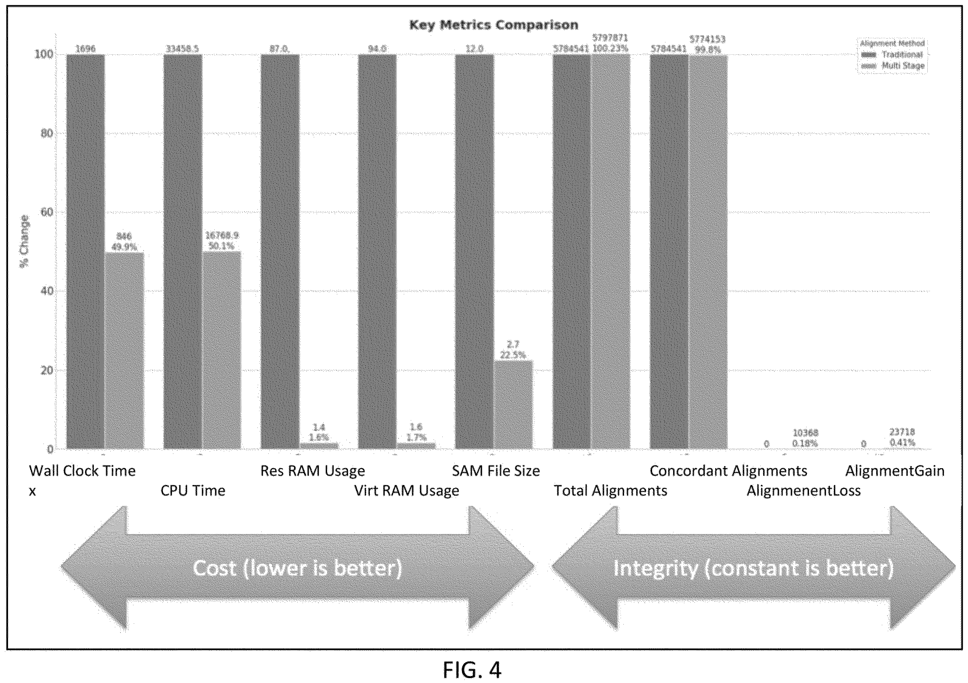

FIG. 4 compares key cost/performance metrics between traditional BWA (blue) and v0.4 Multistage Aligner (orange) executing on vhmem hardware of RSS-SC1 cluster to process San Jose's test case 20180503_uzuki_000001_WSK18R04C8_L03. Cost (first five bars) are drastically reduced, while integrity (last four bars) are equivalent.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

In addition to the claimed subject matter in the appended claims, the following statements may serve as basis for additional claims in this application.
1. A method for aligning sequence reads to a reference sequence, the method comprising:
   aligning a first set of sequence reads from an entire population of sequence reads to a reference sequence with a Burrows-Wheeler transform using a first seed length, wherein the first seed length is selected based on an error rate of the sequence reads;
   masking the first set of sequence reads such that the entire population of sequence reads comprises a subset of masked sequence reads and unmasked sequence reads;
   aligning a second set of sequence reads from the unmasked sequence reads to the reference sequence with the Burrow-Wheeler transform using a second seed length, wherein the second seed length is smaller than the first seed length; and
   determining an alignment of the sequence reads to the reference sequence based on the first set of sequence reads and the second set of sequence reads.
2. The method of statement 1, further comprising iteratively masking and aligning additional sets of sequence reads with each subsequent set of reads having a smaller seed length, and determining the alignment of the sequence reads with the additional sets of sequence reads.
3. The method of statement 1, wherein the first seed length is less than 10 bases.
4. The method of statement 1, wherein the first seed length is less than 5 bases.
5. The method of statement 1, wherein the first seed length is 4 bases.
6. The method of statement 1, wherein the error rate of the sequence reads is at least 5%.
7. The method of statement 1, wherein the error rate of the sequence reads is at least 10%.
8. The method of statement 1, wherein the error rate of the sequence reads is at least 15%.
9. The method of statement 1, wherein the sequence reads are sequenced from a plurality of concatamers, wherein each concatamer is formed of oligonucleotide sequences that have been joined together, wherein the oligonucleotide sequences correspond to a plurality of loci from a set of chromosomes.
10. The method of statement 9, wherein the set of chromosomes comprises chromosome 13, 18, 22, X, and Y.
11. The method of statement 9, wherein the set of chromosomes is selected from the group consisting of chromosome 13, 18, 22, X, and Y.
12. The method of statement 9, further comprising calculating a frequency each loci is found in the sequence reads.
13. A method for aligning sequence reads to a reference sequence, the method comprising:
   aligning a first set of sequence reads from an entire population of sequence reads to a reference sequence with a Burrows-Wheeler transform using a first set of sensitivity parameters, wherein the first set of sensitivity parameters is selected based on an error rate of the sequence reads;
   masking the first set of sequence reads such that the entire population of sequence reads comprises a subset of masked sequence reads and unmasked sequence reads;
   aligning a second set of sequence reads from the unmasked sequence reads to the reference sequence with the Burrow-Wheeler transform using a second set of sensitivity parameters, wherein the second set of sensitivity parameters results in a higher sensitivity than the first set of sensitivity parameters; and
   determining an alignment of the sequence reads to the reference sequence based on the first set of sequence reads and the second set of sequence reads.
14. The method of statement 13, further comprising iteratively masking and aligning additional sets of sequence reads with each subsequent set of reads having a set of sensitivity parameters that results in higher sensitivity, and determining the alignment of the sequence reads with the additional sets of sequence reads.
15. The method of statement 13, wherein the sensitivity parameters are selected from the group consisting of seed generation, chaining and filtering, and thresholding.
16. A computer product comprising a computer readable medium storing a plurality of instructions for controlling a computer system to perform an operation of any of the methods above.
17. A system comprising:
   the computer product of statement 16; and
   one or more processors for executing instructions stored on the computer readable medium.
18. A system comprising means for performing any of the above methods.
19. A system comprising one or more processors configured to perform any of the above methods.
20. A system comprising modules that respectively perform the steps of any of the above methods.

## Claims

1. A method for aligning sequence reads to a reference sequence, the method comprising:
aligning a first set of sequence reads from an entire population of sequence reads to a reference sequence with a Burrows-Wheeler transform using a first set of sensitivity parameters, wherein the first set of sensitivity parameters is selected based on an error rate of the sequence reads;
masking the first set of sequence reads such that the entire population of sequence reads comprises a subset of masked sequence reads and unmasked sequence reads;
aligning a second set of sequence reads from the unmasked sequence reads to the reference sequence with the Burrow-Wheeler transform using a second set of sensitivity parameters, wherein the second set of sensitivity parameters results in a higher sensitivity than the first set of sensitivity parameters; and
determining an alignment of the sequence reads to the reference sequence based on the first set of sequence reads and the second set of sequence reads.

2. The method of claim 1, further comprising iteratively masking and aligning additional sets of sequence reads with each subsequent set of reads having a set of sensitivity parameters that results in higher sensitivity, and determining the alignment of the sequence reads with the additional sets of sequence reads.

3. The method of claim 1, wherein the sensitivity parameters are selected from the group consisting of seed generation, chaining and filtering, and thresholding.

4. A computer product comprising a computer readable medium storing a plurality of instructions for controlling a computer system to perform an operation of any of the methods above.

5. A system comprising:
the computer product of claim 4; and
one or more processors for executing instructions stored on the computer readable medium.

6. A system comprising means for performing any of the above methods.

7. A system comprising one or more processors configured to perform any of the above methods.

8. A system comprising modules that respectively perform the steps of any of the above methods.
